# EUROPÄISCHE PATENTANMELDUNG

(11) **EP 2 055 306 A1**
(43) Veröffentlichungstag der Anmeldung: **06.05.2009**
(21) Anmeldenummer: 07021465.5
(22) Anmeldetag: 05.11.2007
(51) Int. Cl.: A61K 31/565, A61K 31/57, A61P 15/16

(54) **Verwendung eines Gestagens in Kombination mit einem Estrogen und einem oder mehreren pharmazeutisch annehmbaren hilfsstoffen/Trägern zur laktosefreien oralen Kontrazeption**

(71) Anmelder: Bayer Schering Pharma AG, 13353 Berlin (DE)
(72) Erfinder: Fricke, Sabine, 07749 Jena (DE); Pfeifer, Manuela, 07745 Jena (DE); Claußen, Claus, 07743 Jena (DE); Ladwig, Ralf, 07747 Jena (DE); Bürglen, Beate, 07743 Jena (DE)
(74) Vertreter: Cramer, Eva-Maria

(57) **Zusammenfassung**

Gestagene, bevorzugt Dienogest, Chlormadinonacetat oder Levornogetrel in Kombination mit Estrogenen, wie beispielsweise Ethinylestradiol, 17βestradiol oder Estradiolvalerat und einem oder mehreren pharmazeutisch annehmbaren Hilfsstoffen/ Trägern. Es wird eine Möglichkeit zur Verbesserung der Prophylaxe der Laktoseintoleranz in einem eventuell beitragenden Faktor gegeben, auch im Hinblick auf aufwendige Untersuchungen zur Laktoseintoleranz.

Die Erfindung ist auch zur Langzeitanwendung geeignet.

## Beschreibung

Die Erfindung betrifft die Verwendung eines Gestagens in Kombination mit einem Estrogen und einem oder mehreren pharmazeutisch annehmbaren Hilfsstoffen/ Trägern zur Herstellung eines pharmazeutischen Präparates zur laktosefreien oralen Kontrazeption. Es wird eine Möglichkeit zur Verbesserung der Prophylaxe der Laktoseintoleranz in einem eventuell beitragenden Faktor gegeben, auch im Hinblick auf aufwendige Untersuchungen zur Laktoseintoleranz.

### Stand der Technik

Laktoseintoleranz, bezeichnet auch als Milchzuckerunverträglichkeit, ist eine Erkrankung , die primär bei ca. 10 Mio. Menschen in Deutschland vorkommt. Typische Beschwerdebilder der Laktoseintoleranz sind je nach Schweregrad Bauchschmerzen, Völlegefühl, Blähungen, Übelkeit und Durchfall. Man unterscheidet grundsätzlich in genetisch bedingter Laktoseintoleranz , beispielsweise durch angeborenen Enzymdefekt, oder erwobener Laktoseintoleranz, deren Ursache noch weitgehend unbekannt ist. Zur Diagnose der Laktoseintoleranz muss ein H2-Atemtest bzw. eine Dünndarmbiopsie durchgeführt werden. Laktose, ein Disaccharid und das Kohlehydrat der Milch, wird mittels Laktase im Dünndarm in die Einzelzucker gespalten, danach in der Mukosazelle aufgenommen und im Blut weitertransportiert. Gelangt nun der Milchzucker auf Grund fehlender Laktase oder deren verminderter Aktivität ungespalten bzw. teilweise ungespalten in den Dickdarm, so wird der Milchzucker hier durch die angesiedelten Bakterien zu Milch- und Essigsäure, Kohlendioxid, Wasserstoff und Methan zersetzt. In Folge entsteht ein osmotischer Druck, welcher eine verstärkte Darmperistaltik mit Durchfall bewirkt. Gleichzeitig führen die entstandenen Gase im Darm zu Blähungen oder Krämpfen. Je nach Schweregrad der Laktoseintoleranz wird den Betroffenen eine laktosefreie bzw. laktosearme Ernährung (< 3mg Laktose/Tag) angeraten. Der gesunde Erwachsene nimmt täglich 20 bis 30 g Laktose in einer vollwertigen Nahrung auf. 100 g Kuhmilch entsprechen ca. 5 g Laktose. Auch verschiedene Brotsorten und Backwaren, Wurstsorten, Butter und Magarine, Schokolade und Süßstofftabletten enthalten technologisch bedingt Laktose. Therapieempfehlungen sind auch laktasehaltige Enzympräparate, welche gleichzeitig mit der Nahrungsaufnahme einzunehmen sind, um die Milchzuckerspaltung anzuregen.

US 6,881,428 offenbart die Herstellung laktosefreier Milch , wobei der Milch ein Enzym (Laktase) zugesetzt wird.

Aus der pharmazeutischen Technologie ist bekannt, orale Kontrazeptiva (OC) ausschließlich auf Laktosebasis herzustellen. Laktose als Füllstoff in den Formulierungen besitzt aufgrund seiner herausragenden Eigenschaften besondere Eignung. Die entstehenden Formulierungen zeichnen sich durch Festigkeit, guten Zerfall und gute Stabilität aus. Die Steroidhormone, einzeln oder in Kombination sind in einem Laktosegranulat sehr günstig auf die einzelnen Korngrößenklassen verteilt. Entmischungen des Granulats, die sich in einer ungenügenden Gleichverteilung im Gehalt der Wirkstoffe in den Tablettenkernen bemerkbar machen würden, sind hier kaum bekannt. Man kann davon ausgehen, dass bei einem OC auf Laktosebasis auch im sehr niedrigen Dosisbereich (mindestens 15 µg Ethinylestradiol pro Einheit) der Wirkstoff gleichmäßig verteilt vorliegt. Laktose selbst lässt sich gut granulieren und das Granulat problemlos zu Tabletten verarbeiten. Eine Direkttablettierung, die mit spezieller Laktose denkbar wäre, wird bisher aufgrund von Gehaltsungleichförmigkeiten der Wirkstoffe in den einzelnen Formulierungen nicht in Erwägung gezogen und nicht in der Praxis angewendet. Bisher werden OC mit geringer Wirkstoffdosierung durch Granulierung, anschließende Tablettierung und in den meisten Fällen durch einen Umhüllungsprozess hergestellt.

WO 2005030175 offenbart eine Zusammensetzung mit Norethisteronacetat und Estradiol und Cellulosebindern, wobei die als Beispiel ausgewiesene pharmazeutische Zusammensetzung zu 45 % Laktose enthält.

WO 2005030176 offenbart eine Zusammensetzung mit Gestagenen und Cellulosebindern, wobei die als Beispiel ausgewiesene pharmazeutische Zusammensetzung ebenfalls Norethisteronacetat und Estradiol auch zu 45 % Laktose enthält.

### Darstellung der Erfindung

Aufgabe der Erfindung ist es, eine Möglichkeit zu finden, für Patientinnen mit Laktoseintoleranz bzw. für Frauen, welche bisher ihre Laktoseintoleranz noch nicht erkannt haben, ein orales Kontrazeptivum zu realisieren. Damit soll gleichzeitig eine Möglichkeit zur Verbesserung der Prophylaxe der Laktoseintoleranz in einem eventuell beitragenden Faktor gegeben werden, auch im Hinblick auf aufwendige Untersuchungen zur Laktoseintoleranz.

Es wurde nun gefunden, dass die Aufgabe erfindungsgemäß gelöst wird durch die Verwendung in Kombination mit Estrogenen und einem oder mehreren pharmazeutisch annehmbaren Hilfsstoffen/ Trägern zur Herstellung eines pharmazeutischen Präparates zur laktosefreien oralen Kontrazeption. Vorzugsweise werden als Gestagen Dienogest in der Dosierung von 2.0 mg oder 1.5 mg oder Chlormadinonacetat oder Levonorgestrel in äquivalenter Dosierung und als Estrogen Ethinylestradiol in der Dosierung von 0.030 mg oder 0.020 mg oder 0.015 mg oder Estradiol oder Estradiolvalerat in äquivalenter Dosis verwendet. Das Ethinylestradiol kann auch als Clathrat vorliegen.

Es sind auch weitere Gestagene in der erfindungsgemäßen Lösung denkbar. Die Herstellung der erfindungsgemäßen laktosefreien oralen Kontrazeptiva erfolgt auf Cellulosebasis. Die Formulierungen werden durch Granulierung, Tablettierung oder oft durch Überziehen hergestellt.

Eine bisher nicht praktizierte Herstellungsweise für niedrig dosierte OC's ist die Anwendung von Cellulosen als Füllstoff und die Granulierung der Wirkstoffe mit Bindemitteln. Als Bindemittel wird vorzugsweise Hydoxypropylcellulose (HPC) 1 bis 5% bezogen auf die Kernmasse eingesetzt. Aber auch Hypromellose oder Maltodextrin oder Gelatine oder Stärkekleister können als Bindemittel verwendet werden. Dies wurde bisher nicht in Erwägung gezogen, da bei den im Korngrößenspektrum sehr begrenzten Cellulosen vor allem Probleme bei der Wirkstoffverteilung auftraten.

Die Aufgabe wurde erfindungsgemäß auch dadurch gelöst, dass durch Auswahl und ggf. Kombination verschiedener Arten mikrokristalliner Cellulose (MCC), die sich in Schüttdichte, Partikelgröße und Feuchtigkeit unterscheiden wie Avicel PH 101 oder Avicel PH 102 oder Avicel PH 112 oder Kombinationen aus MCC und dibasisches Calciumphosphat oder Mannitol bei gleichzeitiger optimaler Wahl des Verhältnisses Bindemittel zur Gesamtmasse der Formulierung (1-5% zu 100%) überraschenderweise eine Gleichverteilung der meist sehr niedrig dosierten Gestagene und Estrogene in den einzelnen Granulatfraktionen und folgend in der Tablette erzielt wurde. Im Fall des besonders niedrig dosierten EE (minimal sind zur Zeit 15 µg pro Tablette möglich) kann der Wirkstoff zur besseren Verteilung als ethanolische Ethinylestradiolllösung während des Granulierprozesses in das Wirbelbett gesprüht werden.

### Ausführungsbeispiele

### Beispiel 1

Wirkstoffkombination:
2.0 mg Dienogest (DNG)/ 0.030 mg Ethinylestradiol (EE)

### Beispiel 2

Wirkstoffkombination:
2.0 mg Chlormadinonacetat (CMA)/ 0.030 mg EEI

### Beispiel 3

Wirkstoffkombination:
0.125 mg Levonorgestrel (LNG)/ 0.030 mg EE

### Beispiel 4

1.5 mg DNG/ 0.015 mg EE, davon 0.825 mg DNG und 0.015 mg EE als Filmbestandteil

Die detailierte Zusammensetzung der Ausführungsbeispiele ist in Tabelle 1 beschrieben.
In Tabelle 2 ist der Vergleich verschiedener Celluloserezepturen hinsichtlich Wirkstoffverteilung im Granulat und Tablettenkernen in Abhängigkeit vom Cellulosetyp und Menge Bindemittel beschrieben.
Gleichverteilung der Wirkstoffe wird über die Bestimmung der Wirkstoffgehälter in den einzelnen Siebfraktionen (Feinkorn, Mittelkorn, Grobkorn) und über die Bestimmung des CUT der Tablettenkerne über den Tablettierprozess (Anfang, Mitte, Ende) angegeben.

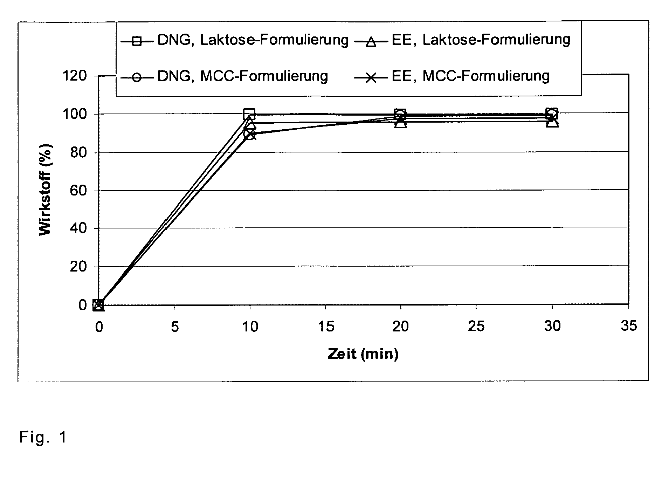

Abbildung 1 zeigt die Freisetzungsdiagramme der cellulosehaltigen Kombination ( 2.0 mg DNG und 0.30 mg EE) im Vergleich zur laktosehaltigen Kombination ( 2.0 mg DNG und 0.030 mg EE), wie mit dem Dissolutiontest in der Freisetzungsapparatur: Paddle unter Verwendung von Wasser mit 37 °C als Dissolutionmedium und 100 U/Min als Rührgeschwindigkeit bestimmt. Es ist ersichtlich, dass die laktosefreie, cellulosehaltigen Wirkstoffkombination in gleicher Art freisetzt wie die laktosehaltigen Wirkstoffkombination.

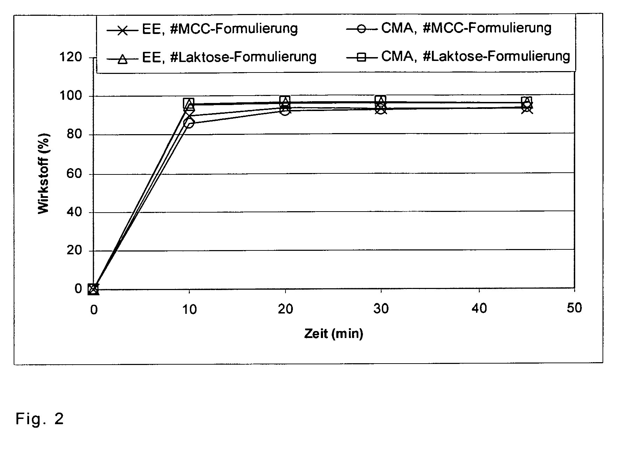

Abbildung 2 zeigt die Freisetzungsdiagramme der cellulosehaltigen Kombination ( 2.0 mg CMA und 0.30 mg EE) im Vergleich zur laktosehaltigen Kombination ( 2.0 mg CMA und 0.030 mg EE), wie mit dem Dissolutiontest in der Freisetzungsapparatur: Paddle unter Verwendung von Natriumdodecylsulfat mit 37 °C als Dissolutionmedium und 75 U/Min als Rührgeschwindigkeit bestimmt. Es ist ersichtlich, dass die laktosefreie, cellulosehaltigen Wirkstoffkombination in gleicher Art freisetzt wie die laktosehaltigen Wirkstoffkombination.

**Tabelle 1**

| **Formulierung (Kern)** | **1** | **2** | **3** | **4 (MR)*** |
|---|---|---|---|---|
| **Wirkstoff** | 2 mg DNG 0,03 mg EE | 2 mg CMA 0,03 mg EE | 0,125 mg LNG 0,03 mg EE | 0,675 mg DNG |
| **Füllstoff** | 43,52 mg MCC | 43,52 mg MCC | 45,395 mg MCC | 58,425 mg MCC |
| **Matrixbildner** | ----- | ----- | ----- | 9 mg Hypro mellose |
| **Sprengmittel** | 1,95 mg Cros car-mellose Natrium | 1,95 mg Cros car-mellose Natrium | 1,95 mg Cros car-mellose Natrium | 15 mg Mais stärke |
| **Bindemittel** | 2 mg Hydroxy propylcellulo se | 2 mg Hydro xypropylcellu lose | 2 mg Hydro xypropylcellu lose | 6 mg Malto-dextrin |
| **Gleitmittel** | 0,5 mg Mag nesium-stearat | 0,5 mg Mag nesium-stearat | 0,5 mg Mag nesium-stearat | 0,9 mg Mag nesium-stearat |

| | | | | |
|---|---|---|---|---|
| *0,825 mg DNG und 0,015 mg EE werden zusätzlich als Filmbestandteile in einer Wirkstoffschicht aufgebracht | | | | |

**Tabelle 2**

| | | **Charg.-Nr. 550907** | **Charg.-Nr. 621007** | **Charg.-Nr. 631007** |
|---|---|---|---|---|
| **Zusammensetzung** | | CMA 2 mg | CMA 2 mg | CMA 2 mg |
| | | EE 0,03 mg | EE 0,03 mg | EE 0,03 mg |
| | | Avicel PH 102 | Avicel PH 102 | Avicel PH 101 |
| | | 44,52 mg | 28,52 mg | 36,02 mg |
| | | HPC 1 mg | Avicel PH 112 | Avicel PH 112 |
| | | Croscarmello se Na 1,95 mg | 15 mg HPC 2 mg | 7,5 mg HPC 2 mg |
| | | Mg-sterat 0,5 mg | Croscarmel lose Na 1,95 | Croscarmel lose Na 1,95 |
| | | | mg | mg |
| | | | Mg-sterat 0,5 | Mg-sterat 0,5 |
| | | | mg | mg |
| **Verteilung Siebfraktionen** | Feinkorn | CMA 125% | CMA 90,5% | CMA 99,4% |
| | | EE 148 % | EE 104,9 % | EE 107,7 % |
| | Mittelkorn | CMA 92% | CMA 114,9% | CMA 97,3% |
| | | EE 93% | EE 118,5% | EE 96,9% |
| | Grobkorn | CMA 79 % | CMA 125,9 % | CMA 111,9% |
| | | EE 52 % | EE 116 % | EE 102,5 % |
| **CUT Kerne (AV-Wert) Soll: < 15** | Anfang | CMA 2,75 | CMA 3,89 | CMA 2,27 |
| | | EE 3,78 | EE 4,20 | EE 9,65 |
| | Mitte | CMA 4,59 | CMA 7,15 | CMA 3,74 |
| | | EE 5,33 | EE 8,59 | EE 3,01 |
| | Ende | CMA 4,68 | CMA 3,58 | CMA 7,12 |
| | | EE 4,62 | EE 6,71 | EE 9,24 |

## Patentansprüche

1. Verwendung eines Gestagens in Kombination mit einem Estrogen und einem oder mehreren pharmazeutisch annehmbaren Hilfsstoffen/ Trägern zur Herstellung eines pharmazeutischen Präparates zur laktosefreien oralen Kontrazeption.

2. Verwendung nach Anspruch 1 **dadurch gekennzeichnet, dass** die Gestagenkomponente 17α-Cyanomethyl-17-β-hydroxyestra-4,9-dien-3on (Dienogest), Chlormadinonacetat oder Levonorgestrel ist.

3. Verwendung nach Anspruch 1 und 2 **dadurch gekennzeichnet, dass** die Estrogenkomponente ein synthetisches Estrogen oder ein natürliches Estrogen bzw. dessen Ester ist.

4. Verwendung nach Anspruch 1, 2 oder 3, **dadurch gekennzeichnet, dass** das synthetische Estrogen Ethinylestradiol und das natürliche Estrogen 17β-Estradiol (Estradiol) oder Estradiolvalerat ist.

5. Verwendung nach Anspruch 1, 2 ,3 oder 4, **dadurch gekennzeichnet, dass** die tägliche Gestagendosis gleich oder kleiner 2 mg Dienogest oder eine äquivalente Menge an Chlormadinonacetat oder Levonorgestrel beträgt.

6. Verwendung nach Anspruch 1, 2 ,3, 4 oder 5, **dadurch gekennzeichnet, dass** die tägliche Gestagendosis 2 mg oder 1.5 mg Dienogest oder eine äquivalente Menge an Chlormadinonacetat oder Levonorgestrel beträgt.

7. Verwendung nach Anspruch 1, 2 ,3, 4, 5 oder 6, **dadurch gekennzeichnet, dass** die tägliche Estrogendosis gleich oder kleiner 0.030 mg Ethinylestradiol oder eine äquivalente Menge an Estradiol oder Estradiolvalerat beträgt.

8. Verwendung nach Anspruch 1, 2 ,3, 4, 5, 6 oder 7, **dadurch gekennzeichnet, dass** die tägliche Estrogendosis 0.030 mg oder 0.020 mg oder 0.015 mg Ethinylestradiol oder eine äquivalente Menge an Estradiol oder Estradiolvalerat beträgt.

9. Verwendung nach Anspruch 1, 2 ,3, 4, 5, 6, 7 oder 8, **dadurch gekennzeichnet, dass** das pharmazeutische Präparat hergestellt wird für die Anwendung von mindestens 21 täglichen Dosiseinheiten und von maximal 7 täglichen einnahmefreien oder placebohaltigen Dosiseinheiten des pharmazeutischen Präparates mit einem oder mehreren pharmazeutisch annehmbaren Hilfsstoffen/ Trägern.

10. Verwendung nach Anspruch 1, 2 ,3, 4, 5, 6, 7, 8 oder 9, **dadurch gekennzeichnet, dass** das pharmazeutische Präparat hergestellt wird für die Anwendung in 21, 22, 23, 24 oder 25 Tagesdosiseinheiten des pharmazeutischen Präparates und in 7, 6, 5, 4 oder 5 einnahmefreien oder placebohaltigen Tagesdosiseinheiten, so dass die Gesamtzahl an Zyklustagen 28 beträgt.

11. Verwendung nach Anspruch 1, 2, 3, 4, 5, 6, 7, 8, 9 oder 10, **dadurch gekennzeichnet, dass** das pharmazeutische Präparat hergestellt wird für die Anwendung von mindestens nx21 täglichen Dosiseinheiten des pharmazeutischen Präparates mit n gleich 2, 3, 4, 5, 6, 7, 8, 9, 10, 11, 12, 13, 14, 15, 16, und 17 und von maximal 7 täglichen einnahmefreien oder placebohaltigen Dosiseinheiten.

12. Verwendung nach Anspruch 11, **dadurch gekennzeichnet, dass** das pharmazeutische Präparat hergestellt wird für die Anwendung von 3, 4, 5, 6 oder 7 täglichen einnahmefreien oder placebohaltigen Dosiseinheiten.

13. Verwendung nach Anspruch 12, **dadurch gekennzeichnet, dass** das pharmazeutische Präparat hergestellt wird für die Anwendung in 84 Tagesdosieinheiten mit der Kombination von Gestagen und Estrogen und in 7 einnahmefreien oder placebohaltigen Tagesdosiseinheiten, so dass die Gesamtzahl an Zyklustagen pro Jahr 4x(nx21 plus 7) mit n gleich 4 ist.

14. Verwendung nach Anspruch 1, 2, 3, 4, 5, 6, 7, 8, 9, 10, 11, 12 oder 13, **dadurch gekennzeichnet, dass** das pharmazeutische Präparat in Form von Tabletten, Filmtabletten, Dragees, Kapseln oder Pulvern vorliegt.

15. Verwendung nach Anspruch 1, 2, 3, 4, 5, 6, 7, 8, 9, 10, 11, 12, 13 o der 14, **dadurch gekennzeichnet, dass** die Arzneiform eine Filmtablette darstellt, bestehend aus einem Tablettenkern mit einem retardierend freizusetzenden anteiligem Gehalt zum Gesamtgehalt an Gestagen und einem Filmüberzug mit einem nicht retardierend (schnell) freizusetzenden anteiligen Gehalt zum Gesamtgehalt an Gestagen und einem nicht retardierend (schnell) freizusetzenden Gesamtgehalt an Estrogen.

16. Verwendung nach Anspruch 15, **dadurch gekennzeichnet, dass** mindestens 10%, vorzugsweise 30% an Gestagen retardiert nach größer 30 Minuten aus dem Tablettenkern herausgelöst werden, wie mit dem Dissolutiontest unter Verwendung von Wasser mit 37 °C als Dissolutionmedium und 50 U/Min als Rührgeschwindigkeit bestimmt.

17. Verwendung nach Anspruch 15 oder 16, **dadurch gekennzeichnet, dass** das Gestagen Dienogest und das Estrogen Ethinylestradiol ist.
